(19) Europäisches Patentamt
European Patent Office
Office européen des brevets

(11) Veröffentlichungsnummer : **0 072 547**
**B1**

(12) # EUROPÄISCHE PATENTSCHRIFT

(45) Veröffentlichungstag der Patentschrift : **26.03.86**

(51) Int. Cl.⁴ : **C 07 J 5/00**, A 61 K 31/57// C07J21/00, C12P33/08

(21) Anmeldenummer : **82107368.1**

(22) Anmeldetag : **13.08.82**

(54) **Neue 6-alpha-Methylprednisolon-Derivate ihre Herstellung und Verwendung.**

(30) Priorität : **18.08.81 DE 3133081**

(43) Veröffentlichungstag der Anmeldung : **23.02.83 Patentblatt 83/08**

(45) Bekanntmachung des Hinweises auf die Patenterteilung : **26.03.86 Patentblatt 86/13**

(84) Benannte Vertragsstaaten : **AT BE CH DE FR GB IT LI LU NL SE**

(56) Entgegenhaltungen :
**EP-A- 0 054 786**
**DE-A- 2 144 405**
**US-A- 2 897 218**
**US-A- 3 176 032**
**US-A- 3 444 217**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Annen, Klaus, Dr.**
**Seegefelder Strasse 194**
**D-1000 Berlin 20 (DE)**
Erfinder : **Petzoldt, Karl, Dr.**
**Flachsweg 10**
**D-1000 Berlin 38 (DE)**
Erfinder : **Laurent, Henry, Dr.**
**Glambecker Weg 21**
**D-1000 Berlin 28 (DE)**
Erfinder : **Wiechert, Rudolf, Prof. Dr.**
**Petzower Strasse 8A**
**D-1000 Berlin 39 (DE)**
Erfinder : **Hofmeister, Helmut, Dr.**
**Weislingenstrasse 4**
**D-1000 Berlin 28 (DE)**
Erfinder : **Wendt, Hans, Dr.**
**Ernst-Ring-Strasse 14**
**D-1000 Berlin 38 (DE)**

**EP 0 072 547 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 6α-Methylprednisolon-Derivate der allgemeinen Formel I

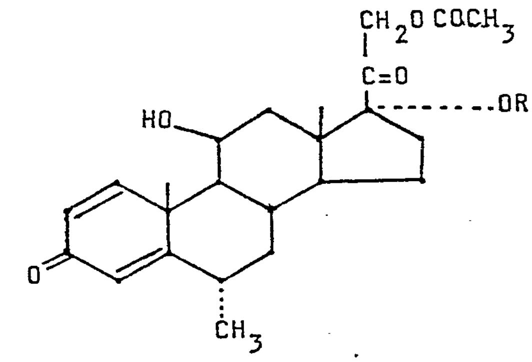

(I)

worin R einen 1-Oxoalkylrest mit 3 bis 6 Kohlenstoffatomen oder einen Benzoylrest darstellt.

Die neuen 6α-Methylprednisolon-Derivate können als 3 bis 6 Kohlenstoffatome enthaltende 1-Oxoalkylgruppen R beispielsweise eine Propionylgruppe, eine Butyrylgruppe, eine Isobutyrylgruppe, eine Valerylgruppe, eine 3-Methylbutyrylgruppe, eine Trimethylacetylgruppe oder eine Hexanoylgruppe tragen.

Es wurde gefunden, daß die Derivate des 6α-Methylprednisolons der Formel I überraschenderweise oft bei topischer Applikation eine signifikant stärkere Wirksamkeit besitzen als die aus der DE-A-21 44 405, der US-A-2,897,218 und der JP-A-79-164 373 vorbekannten Derivate des 6α-Methylprednisolons diese Wirksamkeit ist oft sogar noch signifikant stärker als diejenige fluorierter « Edelkortikoide », wie etwa das 6α,9α-Difluor-11β-hydroxy-16α-methyl-21-valeryloxy-1,4-pregnadien-3,20-dion (=Nerisona) oder das aus der US-A-3,444,217 vorbekannten Betamethason-17-valerat-21-acetat. Die in der US-A-3,176,032 beschriebenen Verbindungen besitzen keine nennenswerte antiinflammatorische Wirksamkeit.

Bei systemischer Applikation sind diese Derivate des 6α-Methylprednisolons überraschenderweise oft schwächer wirksam als die entsprechenden vorbekannten Derivate des 6α-Methylprednisolons.

Die neuen 6α-Methylprednisolon-Derivate der allgemeinen Formel I eigenen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wi. .stoffe mit geeigneten Zusätzen in die gewünschte Applikationsform, wie zum Beispiel : Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die Erfindung betrifft auch ein Verfahren zur Herstellung von 6α-Methylprednisolon-Derivaten der allgemeinen Formel I, welches unter den Bedingungen durchgeführt werden kann, wie sie in den DE-A-26 45 104, 26 45 105 und 23 40 591 und 19 58 549 sowie im US-A-3,383,394 beschrieben sind, und welches dadurch gekennzeichnet ist, daß man in an sich bekannter Weise

a) ein 6α-Methylprednisolon-17-acylat der allgemeinen Formel II

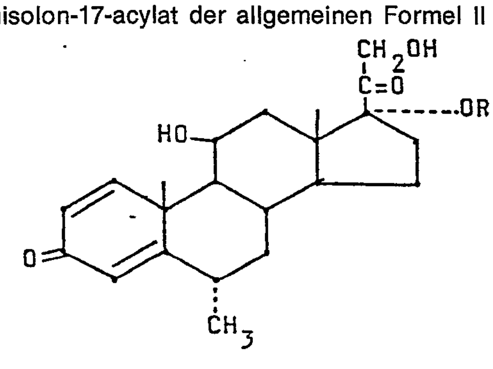

(II)

worin R die obengenannte Bedeutung besitzt, in der 21-Position mit Essigsäure oder einem reaktionsfähigen Derivat derselben verestert, oder

b) ein 6α-Methylprednisolon-21-acylat der allgemeinen Formel III

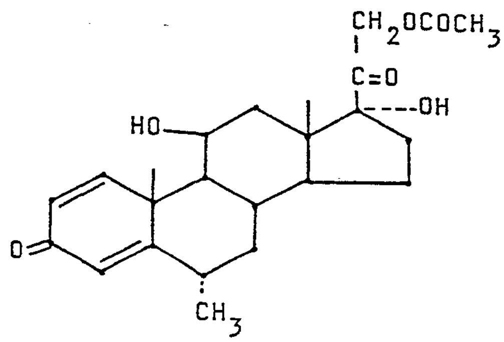

(III)

in der 11-Position mit einer Trialkylsilylverbindung veräthert oder mit einem Derivat einer stark aciden Monocarbonsäure verestert, anschließend die 17-Position mit einem Carbonsäurechlorid oder Carbonsäureanhydrid in Gegenwart von 4-Dimethylaminopyridin acyliert und die Schutzgruppe in der 11-Position abspaltet.

Die nachfolgenden Ausführungsbeispiele dienen zur Erläuterung des erfindungsgemäßen Verfahrens.

### Beispiel 1

a) 10.0 g 11β.17.21-Trihydroxy-6α-methy-1.4-pregnadien-3.20-dion und 1.0 g Pyridiniumtosylat werden in 80 ml Dimethylformamid und 700 ml Benzol gelöst. Bei einer Badtemperatur von 140 °C werden 300 ml Benzol über einen Wasserabscheider abdestilliert, die Lösung kurz auf 80 °C abgekühlt und mit 24 ml Orthopropionsäuretriethylester versetzt. Innerhalb von 20 Minuten destilliert man das restliche Benzol mit den leicht flüchtigen Lösungsmittelkomponenten ab. Nach Zugabe von 12 ml Pyridin wird in Vakuum eingeengt und 17.21-(Ethoxy-propylidendioxy)-11β-hydroxy-6α-methyl-1.4-pregnadien-3.20-dion als Öl erhalten.

b) Eine Suspension obigen Rohprodukts in 300 ml Methanol wird mit einem Gemisch aus 12 ml 0.1 M wäßriger Natriumacetat-Lösung und 108 ml 0.1 N wäßriger Essigsäure 2.5 Stunden bei 100 °C refluxiert. Man engt bis zur Trübung ein, gibt auf Wasser und extrahiert mit viel Essigster. Die organische Phase wird mit Wasser gewaschen, über Natriumsulfat getrocknet und im Vakuum zur Trockne eingedampft. Das Rohprodukt wird an 600 g Kieselgel mit einem Methylenchlorid-Aceton-Gradienten (0-12 % Aceton) gereinigt. Ausbeute 8.9 g 11β.21-Dihydroxy-6α-methyl-17-propionyloxy-1.4-pregnadien-3.20-dion. Schmelzpunkt 214 °C.

c) Eine Lösung von 1.0 g 11β.21-Dihydroxy-6α-methyl-17-propionyloxy-1.4-pregnadien-3.20-dion in 10 ml Pyridin wird mit 5 ml Essigsäureanhydrid 1 Stunde bei 0 °C gerührt. Anschließend gibt man auf eine Eiswasser-Natriumchlorid-Lösung, filtriert den Niederschlag ab und reinigt ihn durch Kristallisation aus Aceton/Hexan.

Man isoliert 790 mg 21-Acetoxy-11β-hydroxy-6α-methyl-17-propionyloxy-1.4-pregnadien-3.20-dion. Schmelzpunkt 138 °C.

### Beispiel 2

a) 10.0 g 6α-Methylprednisolon werden analog Beispiel 2a mit 24 ml Orthobenzoesäuretriethylester zu 17.21-(1-Ethoxy-benzylidendioxy)-11β-hydroxy-6α-methyl-1.4-pregnadien-3.20-dion umgesetzt.

b) Das rohe 17.21-(1-Ethoxy-benzylidendioxy)-11β-hydroxy-6α-methyl-1.4-pregnadien-3.20-dion wird unter den Bedingungen des Beispiels 2b hydrolysiert, aufgearbeitet und gereinigt. Ausbeute 7.5 g 17-Benzoyloxy-11β.21-dihydroxy-6α-methyl-1.4-pregnadien-3.20-dion. Schmelzpunkt 230 °C.

c) 1.9 g 17-Benzoyloxy-11β.21-dihydroxy-6α-methyl-1.4-pregnadien-3.20-dion werden analog Beispiel 1c mit Essigsäureanhydrid umgesetzt und aufgearbeitet. Das Rohprodukt wird an 200 g Kieselgel mit einem Methylenchlorid-Aceton-Gradienten (0-12 % Aceton) gereinigt. Man isoliert 1.8 g 21-Acetoxy-17-benzoyloxy-11β-hydroxy-6α-methyl-1.4-pregnadien-3.20-dion. Schmelzpunkt 258 °C.

### Beispiel 3

0.9 g 17-Butyryloxy-11β.21-dihydroxy-6α-methyl-1.4-pregnadien-3.20-dion werden analog Beispiel 1c mit Essigsäureanhydrid umgesetzt und aufgearbeitet. Das Rohprodukt wird an 40 g Kieselgel mit einem Methylenchlorid-Aceton-Gradienten (0-12 % Aceton) gereinigt. Ausbeute 680 mg 21-Acetoxy-17-butyryloxy-11β-hydroxy-6α-methyl-1.4-pregnadien-3.20-dion. Schmelzpunkt 158 °C.

Beispiel 4

a) Zu einer Suspension von 36.5 g Kupfer (I)-jodid in 730 ml wasserfreiem Tetrahydrofuran werden bei 0 °C unter Argon 200 ml einer 5proz. Lösung von Methyllithium in Ether hinzugetropft. Die gelbe Lösung wird auf — 30 °C abgekühlt und eine Lösung von 29.0 g 11β.17-Dihydroxy-21-isovaleryloxy-6α-methyl-1.4-pregnadien-3.20-dion in 730 ml wasserfreiem Tetrahydrofuran zugesetzt. Man rührt 10 Minuten bei — 25 °C weiter und gibt auf eine wäßrige Ammoniumchloridlösung. Nach der Extraktion mit Methylenchlorid wird die organische Lösung gewaschen, über Natriumsulfat getrocknet und im Vakuum eingedampft. Das Rohprodukt wird an 750 g Kieselgel mit einem Methylenchlorid-Aceton-Gradienten (0-20 % Aceton) gereinigt. Ausbeute 18.2 g 11β.21-Dihydroxy-17-isovaleryloxy-6α-methyl-1.4-pregnadien-3.20-dion. Schmelzpunkt 164 °C.

b) Analog Beispiel 1c werden 1.5 g 11β.21-Dihydroxy-17-isovaleryloxy-6α-methyl-1.4-pregnadien-3.20-dion in 15 ml Pyridin mit 7.5 ml Essigsäureanhydrid umgesetzt, aufgearbeitet und gereinigt. Man isoliert 980 mg 21-Acetoxy-11β-hydroxy-17-isovaleryloxy-6α-methyl-1.4-pregnadien-3.20-dion. Schmelzpunkt 172 °C.

Beispiel 5

a) Unter den Bedingungen des Beispiel 4a werden 3.6 g 11β.17-Dihydroxy-6α-methyl-21-trimethylacetoxy-1.4-pregnadien-3.20-dion zu 780 mg 11β.21-Dihydroxy-6α-methyl-17-trimethylacetoxy-1.4-pregnadien-3.20-dion umgelagert, aufgearbeitet und gereinigt.

b) Wie im Beispiel 1c beschrieben, werden 700 mg 11β.21-Dihydroxy-6α-methyl-17-trimethylacetoxy-1.4-pregnadien-3.20-dion mit Essigsäureanhydrid umgesetzt und aufgearbeitet. Ausbeute 610 mg 21-Acetoxy-11β-hydroxy-6α-methyl-17-trimethylacetoxy-1.4-pregnadien-3.20-dion. Schmelzpunkt 201 °C.

Beispiel 6

a) Analog Beispiel 4a werden 2.0 g 11β.17-Dihydroxy-6α-methyl-21-valeryloxy-1.4-pregnadien-3.20-dion mit Lithium-dimethylcuprat zu 1.8 g 11β.21-Dihydroxy-6α-methyl-17-valeryloxy-1.4-pregnadien-3.20-dion umgelagert, aufgearbeitet und gereinigt.

b) 1.5 g 11β.21-Dihydroxy-6α-methyl-17-valeryloxy-1.4-pregnadien-3.20-dion werden analog Beispiel 1c mit Essigsäureanhydrid zu 1.2 g 21-Acetoxy-11β-hydroxy-6α-methyl-17-valeryloxy-1.4-pregnadien-3.20-dion acetyliert.

Beispiel 7

a) 2.0 g 11β.17-Dihydroxy-21-isobutyryloxy-6α-methyl-1.4-pregnadien-3.20-dion werden analog Beispiel 4a zu 1.3 g 11β.21-Dihydroxy-17-isobutyryloxy-6α-methyl-1.4-pregnadien-3.20-dion umgelagert.

b) 1.0 g 11β.21-Dihydroxy-17-isobutyryloxy-6α-methyl-1.4-pregnadien-3.20-dion werden analog Beispiel 1c mit Essigsäureanhydrid zu 0.7 g 21-Acetoxy-11β-hydroxy-17-isobutyryloxy-6α-methyl-1.4-pregnadien-3.20-dion umgesetzt, aufgearbeitet und gereinigt.

Beispiel 8

a) Eine Lösung von 5.0 g 21-Acetoxy-11β.17-dihydroxy-6α-methyl-1.4-pregnadien-3.20-dion in 25 ml Pyridin wird bei — 15 °C tropfenweise mit 3 ml Trifluoressigsäureanhydrid versetzt und 10 Minuten bei — 10 °C gerührt. Man gibt auf eine Eiswasser-Natriumchloridlösung und filtriert den Niederschlag ab. Den Rückstand nimmt man in Methylenchlorid auf, wäscht neutral und engt nach dem Trocknen über Natriumsulfat in Vakuum ein. Ausbeute 5.4 g 21-Acetoxy-17-hydroxy-6α-methyl-11β-trifluoracetoxy-1.4-pregnadien-3.20-dion.

b) 4.0 g des unter a) erhaltenen Rohprodukts werden in 50 ml Diethylenglykoldimethylether und 6.0 ml Propionsäureanhydrid mit 6.5 g 4-Dimethylaminopyridin 18 Stunden bei Raumtemperatur gerührt. Nach der üblichen Aufarbeitung isoliert man 4.5 g 21-Acetoxy-6α-methyl-17-propionyloxy-11β-trifluoracetoxy-1.4-pregnadien-3.20-dion.

c) 3.5 g 21-Acetoxy-6α-methyl-17-propionyloxy-11β-trifluoracetoxy-1.4-pregnadien-3.20-dion werden in 80 ml Methanol und 4.2 ml Triethylamin 4 Stunden bei Raumtemperatur gerührt. Man reinigt das Rohprodukt an 600 g Kieselgel mit einem Methylenchlorid-Aceton-Gradienten (0-15 % Aceton) und isoliert 2.3 g 21-Acetoxy-11β-hydroxy-6α-methyl-17-propionyloxy-1.4-pregnadien-3.20-dion.

**Patentansprüche**

1. 6α-Methylprednisolon-Derivate der allgemeinen Formel I

(Siehe Formel I Seite 5 f.)

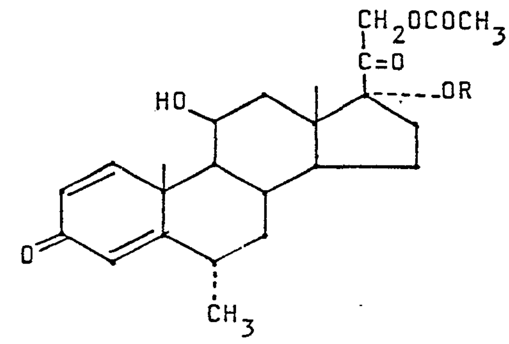

(I)

worin R einen 1-Oxoalkylrest mit 3 bis 6 Kohlenstoffatomen oder einen Benzoylrest darstellt.

2. 21-Acetoxy-11β-hydroxy-17-isobutyryloxy-6α-methyl-1,4-pregnadien-3,20-dion.
3. 21-Acetoxy-11β-hydroxy-6α-methyl-17-trimethylacetoxy-1,4-pregnadien-3,20-dion.
4. 21-Acetoxy-11β-hydroxy-6α-methyl-17-propionyloxy-1,4-pregnadien-3,20-dion.
5. 21-Acetoxy-17-butyryloxy-11β-hydroxy-6α-methyl-1,4-pregnadien-3,20-dion.
6. 21-Acetoxy-11β-hydroxy-6α-methyl-17-isovaleryloxy-1,4-pregnadien-3,20-dion.
7. 21-Acetoxy-11β-hydroxy-17-valeryloxy-6α-methyl-1,4-pregnadien-3,20-dion.
8. 21-Acetoxy-17-benzoyloxy-11β-hydroxy-6α-methyl-1,4-pregnadien-3,20-dion.
9. Pharmazeutische Präparate gekennzeichnet durch den Gehalt an ein oder zwei 6α-Methylprednisolon-Derivaten gemäß Anspruch 1 bis 8.
10. Verfahren zur Herstellung von 6α-Methylprednisolon-Derivaten der allgemeinen Formel I

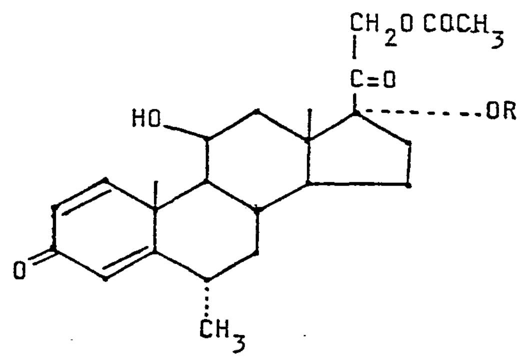

(I)

worin R einen 1-Oxoalkylrest mit 3 bis 6 Kohlenstoffatomen oder einen Benzoylrest bedeutet, dadurch gekennzeichnet, daß man in an sich bekannter Weise

a) ein 6α-Methylprednisolon-17-acylat der allgemeinen Formel II

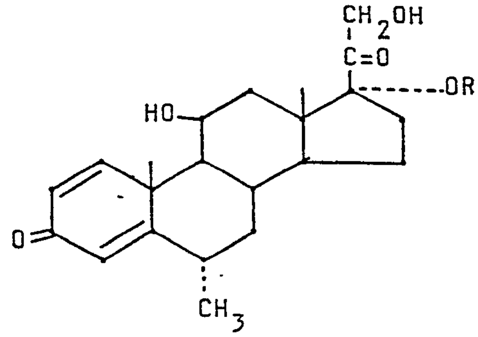

(II)

worin R die obengenannte Bedeutung besitzt, in der 21-Position mit Essigsäure oder einem reaktionsfähigen Derivat derselben verestert, oder

b) ein 6α-Methylprednisolon-21-acylat der allgemeinen Formel III

(Siehe Formel III Seite 6 f.)

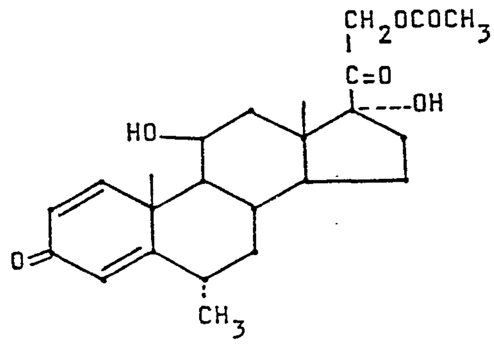

(III)

in der 11-Position mit einer Trialkylsilylverbindung veräthert oder mit einem Derivat einer stark aciden Monocarbonsäure verestert, anschließend die 17-Position mit einem Carbonsäurechlorid oder Carbonsäureanhydrid in Gegenwart von 4-Dimethylaminopyridin acyliert und die Schutzgruppe in der 11-Position abspaltet.

**Claims**

1. 6α-Methylprednisolone derivatives of the general formula I

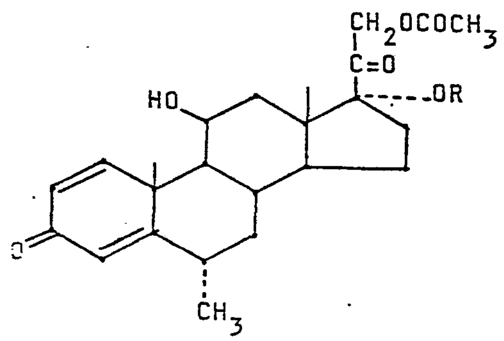

(I)

wherein R is a 1-oxoalkyl group of 3 to 6 carbon atoms or a benzoyl group.

2. 21-Acetoxy-11β-hydroxy-17-isobutyryloxy-6α-methyl-1,4-pregnadiene-3,20-dione.

3. 21-Acetoxy-11β-hydroxy-6α-methyl-17-trimethylacetoxy-1,4-pregnadiene-3,20-dione.

4. 21-Acetoxy-11β-hydroxy-6α-methyl-17-propionyloxy-1,4-pregnadiene-3,20-dione.

5. 21-Acetoxy-17-butyryloxy-11β-hydroxy-6α-methyl-1,4-pregnadiene-3,20-dione.

6. 21-Acetoxy-11β-hydroxy-6α-methyl-17-isovaleryloxy-1,4-pregnadiene-3,20-dione.

7. 21-Acetoxy-11β-hydroxy-17-valeryloxy-6α-methyl-1,4-pregnadiene-3,20-dione.

8. 21-Acetoxy-17-benzoyloxy-11β-hydroxy-6α-methyl-1,4-pregnadiene-3,20-dione.

9. Pharmaceutical compositions characterised in that they contain one or two 6α-methylprednisolone derivatives according to claims 1 to 8.

10. Process for the preparation of 6α-methylprednisolone derivatives of the general formula I

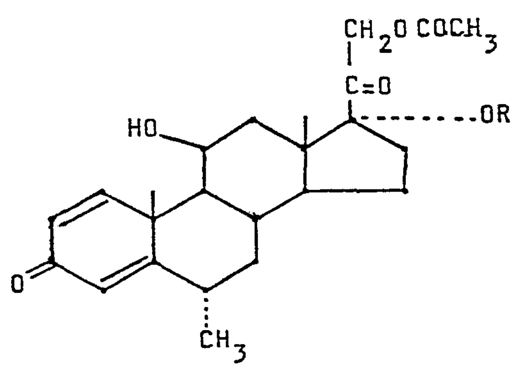

(I)

wherein R is a 1-oxoalkyl group of 3 to 6 carbon atoms or a benzoyl group, characterised in that, in a known manner,

a) a 6α-methylprednisolone-17-acylate of the general formula II

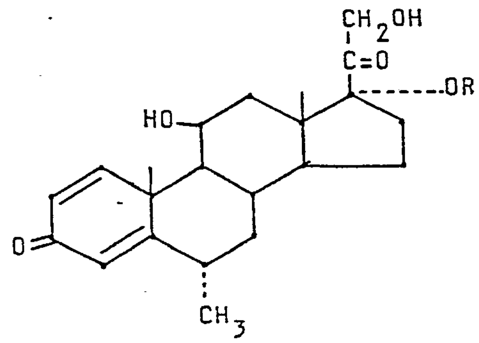

(II)

wherein R has the meaning given above, is esterified in the 21-position with acetic acid or a reactive derivative thereof, or

b) a 6α-methylprednisolone-21-acylate of the general formula III

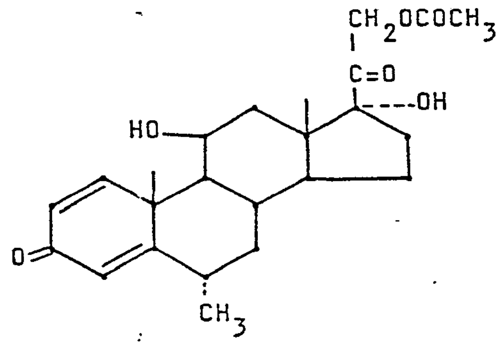

(III)

is estherified in the 11-position with a trialkylsilyl compound or esterified with a derivative of a strongly acidic monocarboxylic acid, following which, the 17-position is acylated with a carbonyl chloride or a carboxylic acid anhydride in the presence of 4-dimethylaminopyridine and the protecting group in the 11-position is removed.

**Revendications**

1. Dérivés de la 6α-méthylprednisolone de formule générale I

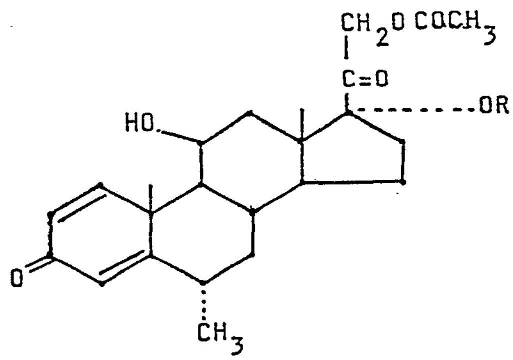

(I)

dans laquelle R représente un radical 1-oxoalkyle en $C_3$ à $C_6$ ou le radical benzoyle.

2. 21-Acétoxy-11β-hydroxy-17-isobutyryloxy-6α-méthyl-1,4-prégnadiène-3,20-dione.
3. 21-Acétoxy-11β-hydroxy-6α-méthyl-17-triméthylacétoxy-1,4-prégnadiène-3,20-dione.
4. 21-Acétoxy-11β-hydroxy-6α-méthyl-17-propionyloxy-1,4-prégnadiène-3,20-dione.
5. 21-Acétoxy-17-butyryloxy-11β-hydroxy-6α-méthyl-1,4-prégnadiène-3,20-dione.
6. 21-Acétoxy-11β-hydroxy-6α-méthyl-17-isovaléryloxy-1,4-prégnadiène-3,20-dione.
7. 21-Acétoxy-11β-hydroxy-17-valéryloxy-6α-méthyl-1,4-prégnadiène-3,20-dione.
8. 21-Acétoxy-17-benzoyloxy-11β-hydroxy-6α-méthyl-1,4-prégnadiène-3,20-dione.
9. Préparations pharmaceutiques contenant une ou deux 6α-méthylprednisolones selon l'une quelconque des revendications 1 à 8.
10. Procédé de préparation de 6α-méthylprednisolones de formule générale I

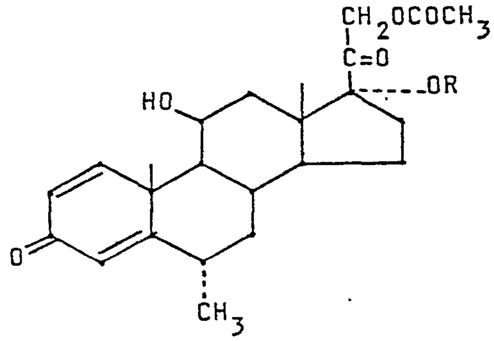

(I)

dans laquelle R représente un radical 1-oxo-alkyle en $C_3$ à $C_6$ ou le radical benzoyle, procédé caractérisé en ce que, de manière connue :

a) on estérifie à la position 21 un 17-acylate de 6α-méthylprednisolone de formule II

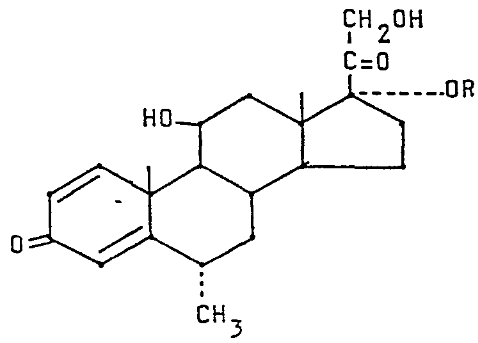

(II)

dans laquelle R a la signification donnée par la formule I, avec de l'acide acétique ou un dérivé réactif de cet acide, ou bien

b) on éthérifie à la position 11 un 21-acylate de 6α-méthylprednisolone de formule III

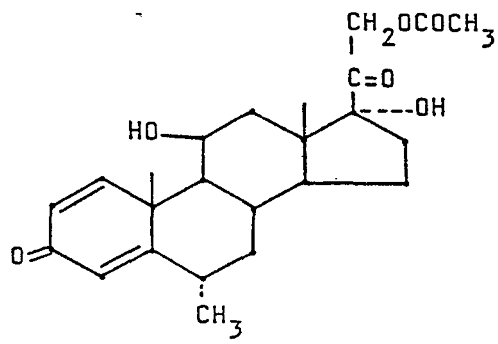

(III)

avec un composé trialkylsilylique ou on l'estérifie avec un dérivé d'un acide monocarboxylique fort, puis on acyle la position 17 avec le chlorure ou l'anhydride d'un acide carboxylique en présence de 4-diméthylamino-pyridine et on élimine le groupe protecteur de la position 11.